(19) Europäisches Patentamt
European Patent Office
Office européen des brevets

(11) EP 0 851 850 B1

(12) EUROPEAN PATENT SPECIFICATION

(45) Date of publication and mention of the grant of the patent:
**12.12.2001 Bulletin 2001/50**

(21) Application number: **96930823.8**

(22) Date of filing: **11.09.1996**

(51) Int Cl.[7]: **C07C 45/00**, C07C 45/45, C07C 259/06

(86) International application number:
**PCT/US96/14565**

(87) International publication number:
**WO 97/10195 (20.03.1997 Gazette 1997/13)**

# (54) SYNTHESIS OF ALPHA-CHLORO OR ALPHA-FLUORO KETONES

SYNTHESE VON ALPHA-CHLOR- ODER ALPHA-FLUORKETONEN

SYNTHESE D'ALPHA-CHLORO OU D'ALPHA-FLUOROCETONES

(84) Designated Contracting States:
**AT BE CH DE DK ES FI FR GB GR IE IT LI LU NL PT SE**

(30) Priority: **15.09.1995 US 3823**
**13.02.1996 GB 9602925**

(43) Date of publication of application:
**08.07.1998 Bulletin 1998/28**

(73) Proprietor: **MERCK & CO., INC.**
**Rahway, New Jersey 07065 (US)**

(72) Inventors:
- **DOLLING, Ulf, H.**
  **Rahway, NJ 07065 (US)**
- **FREY, Lisa, F.**
  **Rahway, NJ 07065 (US)**
- **TILLYER, Richard, D.**
  **Rahway, NJ 07065 (US)**
- **TSCHAEN, David, M.**
  **Rahway, NJ 07065 (US)**

(74) Representative: **Horgan, James Michael Frederic**
**Merck & Co., Inc.,**
**Terlings Park,**
**Eastwick Road**
**Harlow, Essex CM20 2QR (GB)**

(56) References cited:
**US-A- 4 385 177**

- **SYNLETT (SYNLES,09365214);96; (3); PP.225-6, MERCK RES. LAB.;DEP. PROCESS RES.; RATHWAY; 07065; NJ; USA (US), XP002019575 TILLYER R ET AL: "Efficient synthesis of.alpha.-chloro ketones via reaction of organometallic reagents with N-methoxy-N-methylchloroacetamide"**
- **TETRAHEDRON LETTERS, vol. 22, no. 39, 1981, OXFORD GB, pages 3815-3818, XP002019576 S. NAHM ET AL.: "N-Methoxy-N-Methylamides as effective acylating agents"**
- **SYNTHESIS, 1984, STUTTGART DE, pages 37-40, XP002019577 G. FRIOUR ET AL.: "Organomanganous Reagents; IX. Preparation of various halogenated, alkoxylated,aryloxylated, and arylsulfenylated ketones from correspondingly functionalized carboxylic acid chlorides or anhydrides" cited in the application**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

Printed by Jouve, 75001 PARIS (FR)

EP 0 851 850 B1

## Description

This application is directed to an improved process for making α-chloro ketones such as 4-(methylthio)-2-chloroacetophenone, which compounds are intermediates useful in the preparation of certain non-steroidal anti-inflammatory agents. See, for example, WO 95/00501, published January 5, 1995, which is hereby encorporated by reference.

The synthesis of α-chloro ketones such as 4-(methylthio)-2-chloroacetophenone **1**

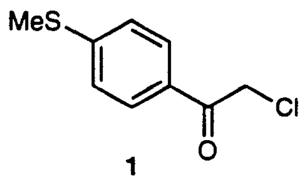

**1**

*via* Friedel-Crafts acylation of thioanisole with chloroacetic acid derivatives, and *via* direct halogenation of 4-(methylthio) acetophenone is possible, but both of these approaches are problematic. For example, while the Friedel-Crafts acylation of thioanisole using acetyl chloride-AlCl3 is an efficient process (>90% yield, 100:1 para:ortho), the use of $AlCl_3$-chloroacetyl chloride is not (<40% yield, 3:1 para:ortho). The situation is not improved by variation of Lewis acid, solvent, or by the use of other chloroacetic acid derivatives. Similarly, the direct halogenation of 4-(methylthio)acetophenone is accompanied by the formation of (usually >10%) di-halogenated ketone, which was difficult to remove by crystallisation.

Moreover, of the many literature protocols for ketone synthesis based on acylation of organometallic reagents, they are generally inapplicable to the synthesis of α-halogenated ketones. See Comprehensive Organic Synthesis, Pergamon 1991, Volume 1, 397 and Friour G., Cahiez G., Normant J. F. *Synthesis 37* **1984,** or organovanadium reagents- Hirao T., Misu D., Yao K., Agawa T., *Tetrahedron Lett.* 929, **1986.** Not surprisingly, the reactions of organometallic reagents **2** (M=Li, MgBr, ZnBr/Cl, MnCl, etc.) with a variety of chloroacetic acid derivatives **3** (e.g. acid chloride, anhydride, imidazolide and nitrile) provided **1** in low yield, due primarily to over-addition and/or enolisation of product by the reagent.

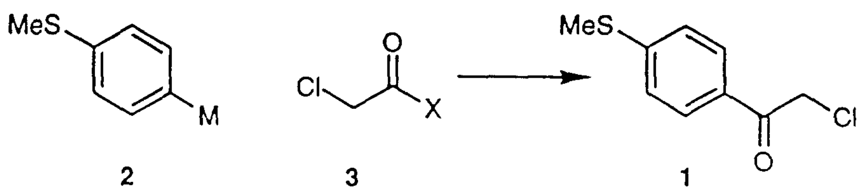

**2** **3** **1**

**2a** M=MgBr **3a** X=Cl

**2b** M=Li **3b** X=NMe(OMe)

Surprisingly, we have found a novel and efficient synthesis of **1**, and other α-chloro ketones *via* reaction of N-methoxy-N-methylchloroacetamide **3b** with organometallic reagents.
Nahm and Weinreb (*Tetrahedron Letters, **22,*** 3815, 1989) describe the synthesis of non-halogenated ketones by reaction of organometallic reagents with N-methoxy-N-methylamides.

## SUMMARY OF THE INVENTION

The invention is directed to a process for making α-chloro or α-fluoro ketones, such as 4-(methylthio)-2-chloroacetophenone, which compounds are intermediates useful in the preparation of non-steroidal anti-inflammatory agents.

## DETAILED DESCRIPTION OF THE INVENTION

The invention encompasses an improved process for making α-chloro ketones, or α-fluoro ketones, including 4-(methylthio)-2-chloroacetophenone, which compounds are intermediates useful in the preparation of non-steroidal anti-inflammatory agents.

In one embodiment, the invention is directed to a process of making an α-chloro or fluoro ketone of the formula I

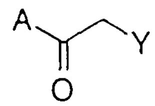

I

wherein Y is chloro or fluoro and A is selected from optionally substituted phenyl, 3,4-dimethoxyphenyl, benzofuranyl, furanyl, imidazolyl, indolyl, isothiazolyl, pyrazinyl, pyrazolyl, pyridyl, pyrimidyl, pyrrolyl, thiazolyl, thienyl, triazolyl, $C_{1-10}$alkyl, $C_{2-3}$alkenyl and $C_{2-3}$alkynyl, wherein the substituent is selected from F, Cl, Br, methylthio, -OH, methoxy, aminothio, $C_{1-3}$alkyl, $C_{2-3}$alkenyl and $C_{2-3}$alkynyl; or A is 3,4-methylenedioxyphenyl; comprising:

(a) reacting an organometallic reagent of formula II

A-M

II

wherein M is Li or Mg Br,
with a compound of formula III

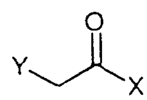

III

wherein X is $-NR(OR^1)$
R and $R^1$ are each independently linear or branched $C_{1-3}$ alkyl, or $C_{3-6}$cycloalkyl, optionally substituted with $C_{1-3}$alkoxy, phenyl, or substituted phenyl, wherein the substituent on the phenyl is selected from F, Cl, Br, methylthio, -OH, methoxy, aminothio, and $C_{1-3}$ alkyl
in an organic aprotic solvent, and

(b) reacting, without further purification, the product of step (a) with an aqueous solution of acid G, to yield an organic aprotic solvent phase comprising compound of formula I and an aqueous solvent phase comprising compound of formula III'

$$HNR(OR^1)\bullet G$$

III'

wherein acid G is hydrochloric, hydrobromic, sulfuric, methanesulfonic, toluenesulfonic or phosphoric acid.

In a preferred aspect of this invention Y is chloro.
The aqueous and organic phases may be separated by standard liquid-liquid separation means, such as by decantation or centrifugation.
For purposes of this specification, the acid G is hydrochloric, hydrobromic, sulfuric, methanesulfonic, toluenesulfonic or phosphoric acid.
For purposes of this specification the organic aprotic solvent includes, but is not limited to ethereal solvents including diethyl ether, di-n-butyl and di-isopentyl ethers, anisole, cyclic ethers such as tetrahydropyran, 4-methyl-1,3-dioxane, tetrahydrofurfuryl methyl ether, ethyl ether, furan, and tetrahydrofuran.

For purposes of this specification A is defined to include optionally substituted:

(1) phenyl,
(2) 3,4-dimethoxyphenyl,
(3) benzofuranyl,
(4) furanyl,
(5) imidazolyl,
(6) indolyl,
(7) isothiazolyl,
(8) pyrazinyl,
(9) pyrazolyl,
(10) pyridyl,
(11) pyrimidyl,
(12) pyrrolyl,
(13) thiazolyl,
(14) thienyl,
(15) triazolyl,
(16) $C_{1-10}$ alkyl,
(17) $C_{2-3}$alkenyl
(18) $C_{2-3}$alkynyl,

wherein the substitutent is selected from F, Cl, Br, methylthio, -OH, methoxy, aminothio, $C_{1-3}$ alkyl, $C_{2-3}$alkenyl, $C_{2-3}$alkynyl.

The reaction step (a) is allowed to proceed until substantially complete in 10 min to 3 hr. The molar ratio of formula II to compound of formula III is typically 1:1 or greater (i.e. excess amount of formula II); preferably 1:1 to 1.2: 1. The reaction may be conducted to -30 to 20°C; preferably 0 to 10°C.

The reaction step (b) is allowed to proceed until substantially complete in 5 min. to 3 hr; typically on the order of 15 minutes. The molar ratio of acid to compound of formula III is typically 1:1 or greater (excess acid); preferably 1: 1 to 1.2:1. The reaction may be conducted at -10 to 20°C; preferably 0 to 5°C.

Within this aspect, there is a class of process wherein the compound of formula I is

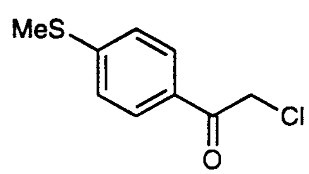

the compound of formula II is

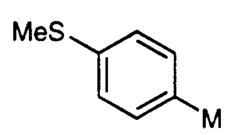

and X is $-NCH_3(OCH_3)$.

There is also disclosed a process of making a compound of formula III

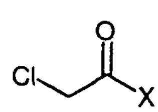

III

wherein X is -NR($OR^1$) wherein R and $R^1$ are described as above, comprising acylating HNR($OR^1$) hydrochloride with chloroacetyl chloride in a two phase mixture of aqueous base and a non-reactive water insoluble organic solvent to yield a compound of formula III.

For purposes of this specification, the base includes both organic bases including pyridine, tri-$C_{1-3}$ alkylamine, and inorganic bases include sodium hydroxide, potassium hydroxide, sodium carbonate or bicarbonate or potassium carbonate or bicarbonate.

For purposes of this specification the non-reactive water insoluble organic solvent includes, but is not limited to toluene, methyl t-butyl ether (MTBE), hexane, heptane, methylene chloride, dichloroethane, dichlorobenzene, monochlorobenzene.

The reaction is allowed to proceed until substantially complete in 5 min. to 1 hr. The molar ratio of HNR($OR^1$) to chloroacetyl chloride is typically 1:1 or greater (i.e. excess chloroacetyl chloride); preferably 1.1:1 to 1.2:1. The reaction is conducted at -10 to 20°C; preferably 0 to 5°C.

A preferred procedure for making a compound of formula III comprises:

separating from the product of reaction step (b) the aqueous solvent phase, said aqueous solvent phase comprising compound of formula III', and

reacting, without further purification, the aqueous solvent phase with chloroacetyl chloride in a bi-phasic mixture of aqueous $K_2CO_3$ and MTBE or toluene, to yield a compound of formula III.

The reaction is allowed to proceed until substantially complete in 5 to 50 minutes. The molar ratio of III' to $K_2CO_3$ is typically 1:2 to 1:3 (i.e. excess base); preferably 1:2.5. The concentration of chloroacetyl chloride in the organic solvent (MTBE or toluene) is 0.2-0.5 M, preferably 0.5 M. The ratio of organic solvent to aqueous solution is 1:1 to 2:1 (excess organic solvent), preferably 1:1. The reaction is conducted to -10 to 5 °C; preferably 0 to 5 °C.

There is also disclosed a practical synthesis of the amide **3b.** A non-aqueous acylation ($Et_3N$, $CH_2Cl_2$) of N,O-dimethylhydroxylamine hydrochloride with chloroacetyl chloride has been reported. See Nuzillard J.-M., Boumendjel G., Massiot G. *Tetrahedron Lett.,* **30** (29) 3779, **1989.** This was accomplished *via* acylation of N,O-dimethylhydroxylamine hydrochloride with chloroacetyl chloride, using a two phase mixture of aq $K_2CO_3$ and toluene/MTBE. The highly pure amide **3b** was isolated in excellent yield (95%) after solvent removal (when MTBE used as solvent). For large scale runs the amide **3b** (prepared using toluene as solvent) was used directly as a solution in toluene (after azeotropic drying) for reactions with organometallic reagents.

Reaction of the amide **3b** with the Grignard reagent **2a** (1M solution in THF, 1.2 equiv., prepared from 4-bromothioanisole) proceeded cleanly and rapidly in THF (reaction complete within 1.5 h at 25°C) to give, after aqueous workup (2N HCl), 4-(methylthio)-2-chloroacetophenone **1** in 90% yield. [Yield determined by quantitative HPLC analysis of the organic layer after workup, compared to a standard solution of chromatographed ketone **1**.] No over addition was observed, and the only significant (>0.5%) by-products were derived from preparation of the Grignard reagent. [By-products from the Grignard formation included the corresponding phenol (oxidation) and biaryl (homocoupling). The former was significantly reduced by de-gassing the reaction solvent and the HCl used for quench.] The crude product was crystallised from toluene-hexane (1:2) to give **1** in 80% yield (>98% purity by HPLC analysis).

A drawback commonly associated with the use of N-methoxy-N-methyl amides for ketone synthesis is the high cost of N,O-dimethylhydroxylamine hydrochloride. Although aqueous workup regenerates N,O-dimethylhydroxylamine (or its hydrochloride salt), there are few, if any, reports concerning its recovery from the aqueous extract. Recycling of this material is now possible *via* direct acylation of N,O-dimethylhydroxylamine in the aqueous extract with chloroacetyl chloride. Thus, after workup (aq HCl) the aqueous extract was reacted with chloroacetyl chloride-$K_2CO_3$ to give the amide **3** cleanly (80% yield based on N,O-dimethylhydroxylamine hydrochloride used initially). This material was re-used in reactions with Grignard reagent **2a** to give **1** without loss in yield or product purity.

**EXAMPLE 1**

Procedure for the preparation of the 4-(methylthio)-2-chloroacetophenone (1)

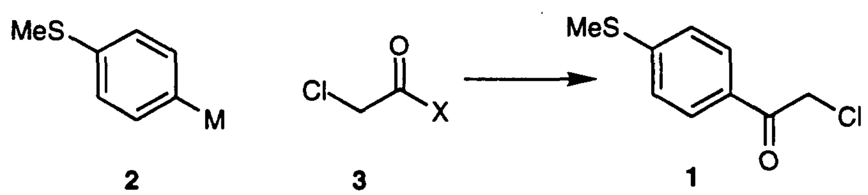

**2** **3** **1**

**2a** M=MgBr **3a** X=Cl

**2b** M=Li **3b** X=NMe(OMe)

A solution of N-methoxy-N-methylchloroacetamide **3b** (26.8 g, 195 mmol) in toluene (250 mL, see ref 9) was diluted with de-gassed THF (530 mL) and the mixture was cooled to 0°C. A solution of the Grignard reagent **2a** (240 mL of a 1M soln in THF, 1.2 equiv.) was added, *via* cannula, over 30 min (solution temperature <5°C) and the mixture was stirred at 25°C for 1.5 h. The thick slurry was transferred, *via* cannula, into cold (0°C), de-gassed aq. 2N HCl (250 mL, 2.5 equiv.) and the layers were separated. The organic layer was washed with brine (100 mL), dried ($MgSO_4$), filtered and concentrated (to approx. 75 mL). Hexanes (150 mL) was added and the mixture was stirred for 2 h. The slurry was filtered, the solid was washed with hexanes and dried to give 32.9 g (80% from N,O-dimethylhydroxylamine hydrochloride) of **1**, a yellow solid.
$^1H$ NMR ($CDCl_3$, 300 MHz) ∂ 2.53 (s, 3H), 4.67 (s, 2H), 7.28 (d, 2 H, *J=8.5* Hz), 7.87 (d, 2H, *J*=8.6 Hz); $^{13}C$ NMR ($CDCl_3$, 75 MHz) ∂ 14.69, 45.77, 125.07, 128.95, 130.35, 147.48, 190.16.

The aqueous layer was added to a biphasic mixture of $K_2CO_3$ (79 g, 570 mmol) in $H_2O$ (200 mL) and MTBE (500 mL) to give a heavy slurry. The vigorously stirred mixture was cooled to -5°C and chloroacetyl chloride (19.6 mL, 246 mmol) was added, over 5 min (solution temperature <1°C). The mixture was warmed to 15°C over 30 min, the layers were separated and the aqueous layer was extracted with MTBE (5x100mL). The combined organic extracts were concentrated to give 22.7 g (80% based on 20 g of N,O-dimethylhydroxylamine hydrochloride used initially) of amide **3b.** This material was identical spectroscopically and by HPLC analysis to **3b** prepared from fresh N,O-dimethylhydroxylamine hydrochloride.

The reactions listed as Examples 2 through 6 (Table I) were conducted using the procedure of Example 1 with appropriate substitution of reagent 2.

**Table 1**

| Example | Reagent | product | yield[1] |
|---|---|---|---|
| 1 | **2a(2b)** | 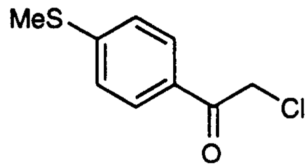 | 90 (92%)[2] |
| 2 | 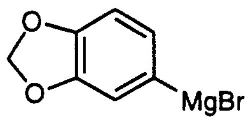 | 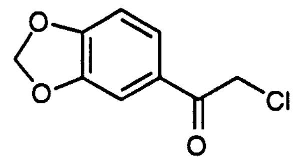 | 83 (76)[2] |
| 3 | 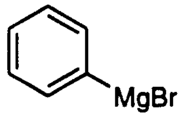 | 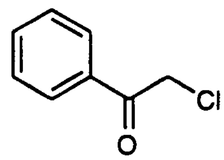 | 92 |
| 4 | 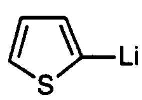 | 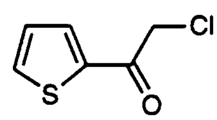 | 87 |
| 5 | $C_8H_{18}MgBr$ | 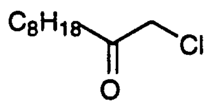 | 95 |
| 6 | 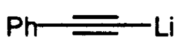 | 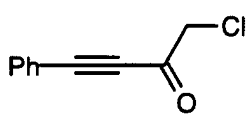 | 86[3] |

1. Isolated yield after chromatography; 2. Yields in brackets correspond to reactions involving organolithium reagents; 3. Reaction carried out at -10°C for 45 min.

**EXAMPLE 2**

A solution of N-methoxy-N-methylchloroacetamide **3b** (1.65 g, 12 mmol) in toluene (15 mL) was diluted with de-gassed THF (27 mL) and the mixture was cooled to 0°C. A solution of the Grignard reagent (14.4 mL of a 1M soln in THF, 1.2 equ, prepared from the reaction of 4-bromo-1,2-(methylenedioxy) benzene with Mg in THF) was added, via cannula, over 30 min (solution temperature <5°C) and the mixture was stirred at 25°C for 2.5 h. The mixture was transferred, via cannula, into cold (0°C), de-gassed aq. 2N HCl (35 mL, 5.8 equ), diluted with tolune (10 mL), and the layers were separated. The organic layer was washed with brine (20 mL), dried ($MgSO_4$), filtered and concentrated to give the product in 83% yield.

A solution of 4-bromo-1,2-(methylenedioxy) benzene (2.2 mL, 18 mmol) in de-gassed THF (50 mL) was cooled to -78°C and n-BuLi (11.3 mL of 1.6M soln, 1.2 equ) was added. After stirring for 50 min at -50°C, a solution of N-methoxy-N-methylchloroacetamide 3b (2.06 g, 15 mmol) in de-gassed THF (14 mL) was slowly added, and the mixture was aged 1 h at -20°C. The mixture was transferred, via cannula, into cold (0°C), de-gassed aq. 2N HCl (37.5 mL, 5.0

equ), diluted with ethyl acetate (20 mL), and the layers were separated. The organic layer was washed with brine (20 mL), dried (MgSO4), filtered and concentrated to give the product in 76% yield. 1H NMR (CDCl3, 300 MHz) ∂4.62 (s, 2H), 6.06 (s, 2H), 6.86 (d, 1H, J=8.1 Hz), 7.41 (d, 1H, J=1.8Hz), 7.54 (m, 1H; 13 C NMR (CDCl3) ∂ 45.76, 102.16, 108.17, 108.26, 125.08, 128.91, 148.49, 152.59, 189.28.

**EXAMPLES 3 TO 5**

Following the procedures of Examples 1 and 2 using the reagents listed in Table 1, the corresponding product was produced.

**EXAMPLE 6**

A solution of phenyl acetylene (2.0 mL, 18 mmol) in de-gassed THF (30 mL) was cooled to -78°C and n-BuLi (11.3 mL of 1.6M soln, 1.5 equ) was added. After stirring for 20 min, the solution was allowed to warm to -20°C. A solution of N-methoxy-N-methylchloroacetamide 3b (1.65 g, 12 mmol) in de-gassed THF (13 mL) was slowly added, and the mixture was aged 15 min at -20°C followed by 45 min at -10°C. The mixture was transferred, via cannula, into cold (0°C), de-gassed aq. 2N HCl (35 mL, 5.8 equ), diluted with ethyl acetate (10 mL), and the layers were separated. The organic layer was washed with brine (20 mL), dried (MgSO4), filtered and concentrated. After silica gel chromatography (97:3 hexanes:ethyl acetate), the compound was isolated in 86% yield. $^1$H NMR (CVDcl3, 300 MHz) ∂4.33 (s, 2H), 7.41 (m, 2H), 7.51 (m, 1H), 7.62 (m, 2H); $^{13}$C NMR (CDCl3) 49.53, 85.55, 95.40, 119.22, 128.79 (2C), 131.46, 133.40 (2C), 178.89.

We have extended this chemistry to the preparation of α-monofluoro ketones *via* reaction of organometallic reagents with N-methoxy-N-methylfluoroacetamide **(5).** For example, phenacyl fluoride **(6)** was prepared in 88% yield *via* reaction of **5** with phenylmagnesium bromide. Many other extensions of this chemistry should be possible.

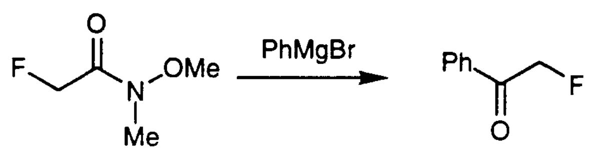


5

6

In summary, we have described a practical synthesis of N-methoxy-N-methylchloroacetamide **3b** and have shown that this compound reacts cleanly with Grignard and organolithium reagents to produce α -monochloro ketones in high yield. The ability to recycle the N,O-dimethylhydroxylamine in a highly efficient manner increases cost effectiveness and makes this procedure particularly attractive for large scale synthesis.

**EXAMPLE 7**

**Preparation of N-methoxy-N-methylchloroacetamide (3b).**

To a cold (0°C), stirred solution of $K_2CO_3$ (62.4 g, 450 mmol) in $H_2O$ (250 mL) was added, successively, N, O-dimethylhydroxylamine hydrochloride (20 g, 205 mmol) and organic solvent (250 mL, toluene or MTBE). The resulting two phase mixture was cooled to -5°C and chloroacetyl chloride (19.6 ml, 246 mmol) was added over 5 min (solution temperature maintained below 0°C). The vigorously stirred mixture was allowed to warm to 15°C over 30 min, the layers were separated, and the aqueous layer was extracted with organic solvent (3x100 mL, toluene or MTBE). The combined organic extracts were concentrated (MTBE used as solvent) to give the amide **3b** (26.8 g, 95%) as a white solid. Alternatively, the combined organic extracts (toluene used as solvent) were concentrated to 250 mL to effect azeotropic drying (water content 100µg/mL) and the solution of **3b** was used directly in reactions with organometallic reagents.

## Claims

**1.** A process of making an α-chloro or fluoro ketone of the formula I

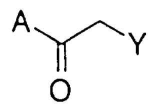

I

wherein Y is chloro or fluoro and A is selected from optionally substituted phenyl, 3,4-dimethoxyphenyl, benzofuranyl, furanyl, imidazolyl, indolyl, isothiazolyl, pyrazinyl, pyrazolyl, pyridyl, pyrimidyl, pyrrolyl, thiazolyl, thienyl, triazolyl, $C_{1-10}$alkyl, $C_{2-3}$alkenyl and $C_{2-3}$alkynyl, wherein the substituent is selected from F, Cl, Br, methylthio, -OH, methoxy, aminothio, $C_{1-3}$alkyl, $C_{2-3}$alkenyl and $C_{2-3}$akynyl; or A is 3,4-methylene dioxy phenyl; comprising:

(a) reacting an organometallic reagent of formula **II**

A-M

**II**

wherein M is Li or MgBr,
with a compound of formula **III**

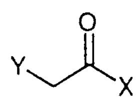

**III**

wherein X is -NR($OR^1$)
R and $R^1$ are each independently linear or branched $C_{1-3}$ alkyl, or $C_{3-6}$cycloalkyl, optionally substituted with $C_{1-3}$alkoxy, phenyl, or substituted phenyl, wherein the substituent on the phenyl is selected from F, Cl, Br, methylthio, -OH, methoxy, aminothio, and $C_{1-3}$ alkyl
in an organic aprotic solvent, and

(b) reacting, without further purification, the product of step (a) with an aqueous solution of acid G, to yield an organic aprotic solvent phase comprising compound of formula I and an aqueous solvent phase comprising compound of formula III'

HNR($OR^1$)•G

**III'**

wherein acid G is hydrochloric, hydrobromic, sulfuric, methanesulfonic, toluenefulfonic or phosphoric acid.

**2.** A process according to Claim 1 wherein Y is chloro.

**3.** A process according to Claim 1 wherein the organic aprotic solvent is selected from the group consisting of diethyl ether, di-n-butyl and di-isopentyl ethers, anisole, tetrahydropyran, 4-methyl-1,3-dioxane, tetrahydrofurfuryl methyl ether, furan, and tetrahydrofuran or a mixture thereof.

**4.** A process according to Claim 1 wherein **A** is an optionally substituted:

(1) phenyl,
(2) 3,4-dimethoxyphenyl,
(3) benzofuranyl,

(4) thienyl,
(5) $C_{1-10}$ alkyl,
(6) $C_{2-3}$alkenyl
(7) $C_{2-3}$alkynyl,

wherein the substitutent is selected from F, Cl, Br, methylthio, -OH, methoxy, aminothio and $C_{1-3}$ alkyl.

**5.** A process according to Claim 1 wherein the compound of formula I is

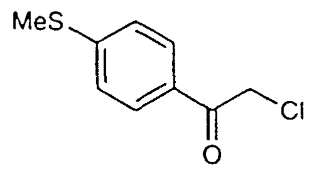

the compound of formula II is

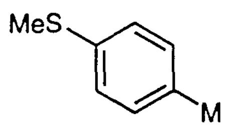

and X is $-NCH_3(OCH_3)$.

**6.** A process according to any previous claim wherein the organic and aqueous solvent phases provided by step (b) are separated and the compound of formula I is isolated from the organic solvent phase.

**7.** The process of claim 2 wherein G is hydrochloric acid, said process further comprising:

separating from the product of reaction step (b) the aqueous solvent phase comprising the compound of formula III'; and
reacting, without further purification, said aqueous solvent phase with chloroacetyl chloride in a two phase mixture of aqueous base and a non-reactive water insoluble organic solvent to yield a compound of formula III wherein Y is chloro.

**8.** The process of claim 7 wherein said aqueous base is potassium carbonate, and said non-reactive water insoluble organic solvent is methyl t-butyl ether or toluene.

**9.** The process of claim 8 wherein said non-reactive water insoluble organic solvent is MTBE, and the compound of formula III wherein Y is chloro is isolated by removal of same.

**10.** The process of claim 8 wherein said non-reactive water insoluble organic solvent is toluene, and the compound of formula III wherein Y is chloro is obtained as a toluene solution which is dried azeotropically.

**11.** A process according to any of claims 7 to 10 wherein the compound of formula III is N-methoxy-N-methylchloroacetamide.

**12.** A process according to any of claims 9 to 11 wherein the compound of formula III wherein Y is chloro is further reacted with an organometallic reagent in a process according to claim 1.

**13.** A process according to claim 1

wherein A is 3,4-methylenedioxyphenyl or phenylethynyl, and

wherein M is Li or MgBr when A is 3,4- methylenedioxyphenyl and M is Li when A is phenylethynyl;

N-methoxy-N-methylchloroacetamide the compound of formula III is ; and the compond of formula III' is:

$$HNCH_3(OCH_3)\bullet G .$$

**Patentansprüche**

1. Ein Verfahren zur Herstellung eines α-Chlor- oder -Fluorketons der Formel I

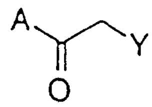

I,

worin Y Chlor oder Fluor ist und A ausgewählt ist aus gegebenenfalls substituiertem Phenyl, 3,4-Dimethoxyphenyl, Benzofuranyl, Furanyl, Imidazolyl, Indolyl, Isothiazolyl, Pyrazinyl, Pyrazolyl, Pyridyl, Pyrimidyl, Pyrrolyl, Thiazolyl, Thienyl, Triazolyl, $C_{1-10}$-Alkyl, $C_{2-3}$-Alkenyl und $C_{2-3}$-Alkinyl, wobei der Substituent ausgewählt ist aus F, Cl, Br, Methylthio, -OH, Methoxy, Aminothio, $C_{1-3}$-Alkyl, $C_{2-3}$-Alkenyl und $C_{2-3}$-Alkinyl, oder A 3,4-Methylendioxyphenyl ist, umfassend:

(a) die Umsetzung eines metallorganischen Reagenzes der Formel II,

A-M

II,

worin M Li oder MgBr ist,
mit einer Verbindung der Formel III

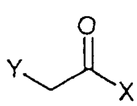

III,

worin X $-NR(OR^1)$ ist,

R und $R^1$ jeweils unabhängig lineares oder verzweigtes $C_{1-3}$-Alkyl oder $C_{3-6}$-Cycloalkyl ist, gegebenenfalls substituiert mit $C_{1-3}$-Alkoxy, Phenyl oder substituiertem Phenyl, wobei der Substituent am Phenyl ausgewählt ist aus F, Cl, Br, Methylthio, -OH, Methoxy, Aminothio und $C_{1-3}$-Alkyl, in einem organischen aprotischen Lösungsmittel, und

(b) die Umsetzung des Produkts von Schritt (a) ohne weitere Reinigung mit einer wäßrigen Lösung von Säure G, um eine organische aprotische Lösungsmittelphase, die eine Verbindung der Formel I enthält, und eine wäßrige Lösungsmittelphase, die eine Verbindung der Formel III'

$$HNR(OR^1)\cdot G$$

III'

enthält, zu ergeben, wobei die Säure G Salz-, Bromwasserstoff-, Schwefel-, Methansulfon-, Toluolsulfon- oder Phosphorsäure ist.

**2.** Ein Verfahren gemäß Anspruch 1, bei dem Y Chlor ist.

**3.** Ein Verfahren gemäß Anspruch 1, bei dem das organische aprotische Lösungsmittel ausgewählt ist aus der Gruppe, bestehend aus Diethylether, Di-n-butyl- und Diisopentylethern, Anisol, Tetrahydropyran, 4-Methyl-1,3-dioxan, Tetrahydrofurfurylmethylether, Furan und Tetrahydrofuran oder einer Mischung davon.

**4.** Ein Verfahren gemäß Anspruch 1, bei dem das Kohlenstoff-Nukleophil ein gegebenenfalls substituiertes:

(1) Phenyl,
(2) 3,4-Dimethoxyphenyl,
(3) Benzofuranyl,
(4) Thienyl,
(5) $C_{1-10}$-Alkyl,
(6) $C_{2-3}$-Alkenyl,
(7) $C_{2-3}$-Alkinyl

ist, wobei der Substituent ausgewählt ist aus F, Cl, Br, Methylthio, -OH, Methoxy, Aminothio und $C_{1-3}$-Alkyl.

**5.** Ein Verfahren gemäß Anspruch 1, bei dem die Verbindung der Formel I

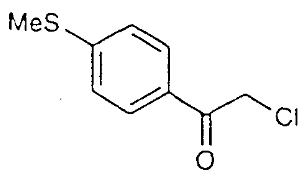

ist,
die Verbindung der Formel II

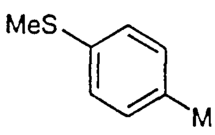

ist
und X $-NCH_3(OCH_3)$ ist.

**6.** Ein Verfahren gemäß irgendeinem vorhergehenden Anspruch, bei dem die durch Schritt (b) zur Verfügung gestellte organische und wäßrige Lösungsmittelphase getrennt werden und die Verbindung der Formel I aus der organischen Lösungsmittelphase isoliert wird.

**7.** Das Verfahren nach Anspruch 2, bei dem G Salzsäure ist und wobei das Verfahren ferner umfaßt:

die Abtrennung der wäßrigen Lösungsmittelphase, welche die Verbindung der Formel III' enthält, von dem Produkt des Reaktionsschrittes (b), und
die Umsetzung der wäßrigen Lösungsmittelphase ohne weitere Reinigung mit Chloracetylchlorid in einer Zweiphasenmischung aus wäßriger Base und einem nichtreaktiven wasserunlöslichen organischen Lösungsmittel, um eine Verbindung der Formel III zu ergeben, worin Y Chlor ist.

**8.** Das Verfahren nach Anspruch 7, bei dem die wäßrige Base Kaliumcarbonat ist und das nichtreaktive wasserunlösliche organische Lösungsmittel Methyl-t-butylether oder Toluol ist.

**9.** Das Verfahren nach Anspruch 8, bei dem das nichtreaktive wasserunlösliche organische Lösungsmittel MTBE ist und die Verbindung der Formel III, worin Y Chlor ist, durch Entfernen derselben isoliert wird.

**10.** Das Verfahren nach Anspruch 8, bei dem das nichtreaktive wasserunlösliche organische Lösungsmittel Toluol ist und die Verbindung der Formel III, worin Y Chlor ist, als eine Toluollösung erhalten wird, welche azeotrop getrocknet wird.

**11.** Ein Verfahren gemäß irgendeinem der Ansprüche 7 bis 10, bei dem die Verbindung der Formel III N-Methoxy-N-methylchloracetamid ist.

**12.** Ein Verfahren gemäß irgendeinem der Ansprüche 9 bis 11, bei dem die Verbindung der Formel III, worin Y Chlor ist, mit einem metallorganischen Reagenz in einem Verfahren gemäß Anspruch 1 weiter umgesetzt wird.

**13.** Ein Verfahren gemäß Anspruch 1, bei dem A 3,4-Methylendioxyphenyl oder Phenylethinyl ist und worin M Li oder MgBr ist, wenn A 3,4-Methylendioxyphenyl ist, und worin M Li ist, wenn A Phenylethinyl ist, bei dem die Verbindung der Formel III N-Methoxy-N-methylchloracetamid ist und die Verbindung der Formel III'

$$HNCH_3(OCH_3) \cdot G$$

ist.

**Revendications**

**1.** Procédé de préparation d'une $\alpha$-chloro- ou fluorocétone de formule 1 :

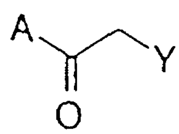

I

dans laquelle Y est le radical chloro ou fluoro, et A est choisi parmi les groupes éventuellement substitués phényle, 3,4-diméthoxyphényle, benzofurannyle, furannyle, imidazolyle, indolyle, isothiazolyle, pyrazinyle, pyrazolyle, pyridyle, pyrimidyle, pyrrolyle, thiazolyle, thiényle, triazolyle, alkyle en $C_{1-10}$, alcényle en $C_{2-3}$ et alcynyle en $C_{2-3}$, où le substituant est choisi parmi F, Cl, Br, les groupes méthylthio, -OH, -méthoxy, aminothio, alkyle en $C_{1-3}$, alcényle en $C_{2-3}$ et alcynyle en $C_{2-3}$ ; ou encore A est le groupe 3,4-méthylènedioxyphényle ; qui comprend :

(a) la réaction d'un réactif organométallique de formule II :

A-M

II

dans laquelle M est Li ou MgBr,
avec un composé de formule III :

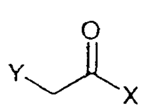

III

dans laquelle X est $-NR(OR^1)$

R et $R^1$ représentent chacun indépendamment de l'autre un groupe alkyle en $C_{1-3}$ à chaîne linéaire ou ramifiée ; ou cycloalkyle en $C_{3-6}$, éventuellement substitué par des substituants alcoxy en $C_{1-3}$, phényle ou phényle substitué, où le substituant du groupe phényle est choisi parmi les groupes F, Cl, Br, méthylthio, -OH, méthoxy, aminothio et alkyle en $C_{1-3}$,

dans un solvant organique aprotique, et

(b) la réaction, sans autre purification, du produit de l'étape (a) avec une solution aqueuse d'un acide G, pour donner une phase de solvant organique aprotique comprenant le composé de formule I et une phase de solvant aqueux contenant le composé de formule III' :

$$HNR(OR^1)\bullet G$$

III'

où l'acide G est l'acide chlorhydrique, bromhydrique, sulfurique, méthane-sulfonique, toluènesulfonique ou phosphorique.

**2.** Procédé selon la revendication 1, dans lequel Y est le groupe chloro.

**3.** Procédé selon la revendication 1, dans lequel le solvant organique aprotique est choisi dans le groupe comprenant le diéthyléther, le di-n-butyléther et le diisopentyléther, l'anisole, le tétrahydropyranne, le 4-méthyl-1,3-dioxanne, l'éther méthylique de tétrahydrofurfuryle, le furanne et le tétrahydrofuranne ou un de leurs mélanges.

**4.** Procédé selon la revendication 1, dans lequel le carbone nucléophile est un groupe éventuellement substitué

( 1 ) phényle,
(2) 3,4-diméthoxyphényle,
(3) benzofurannyle,
(4) thiényle,
(5) alkyle en $C_{1-10}$,
(6) alcényle en $C_{2-3}$,
(7) alcynyle en $C_{2-3}$,

où le substituant est choisi parmi les groupes F, Cl, Br, méthylthio, -OH, méthoxy, aminothio et alkyle en $C_{1-3}$.

**5.** Procédé selon la revendication 1, dans lequel le composé de formule I est

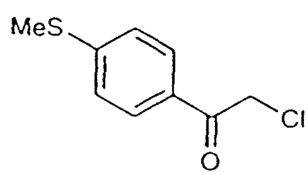

le composé de formule II est

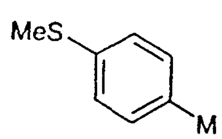

et X est $-NCH_3(OCH_3)$.

**6.** Procédé selon l'une quelconque des revendications précédentes, dans lequel les phases de solvant organique et aqueux obtenues dans l'étape (b) sont séparées, et le composé de formule I est isolé de la phase de solvant organique.

**7.** Procédé selon la revendication 2, dans lequel G est l'acide chlorhydrique, ledit procédé comprenant en outre :

- la séparation, d'avec le produit de l'étape de réaction (b), de la phase de solvant aqueux comprenant le composé de formule III' ; et
- la réaction, sans autre purification, de ladite phase de solvant aqueux avec du chlorure de chloracétyle dans un mélange biphasique d'une base aqueuse et d'un solvant organique non-réactif insoluble dans l'eau, pour donner un composé de formule III dans laquelle Y est le groupe chloro.

**8.** Procédé selon la revendication 7, dans lequel ladite base aqueuse est le carbonate de potassium, et ledit solvant organique non-réactif insoluble dans l'eau est le méthyl-tert-butyléther ou le toluène.

**9.** Procédé selon la revendication 8, dans lequel ledit solvant organique non-réactif insoluble dans l'eau est le MTBE, et le composé de formule III dans laquelle Y est le groupe chloro est isolé par son élimination.

**10.** Procédé selon la revendication 8, dans lequel ledit solvant organique non-réactif insoluble dans l'eau est le toluène, et le composé de formule III dans laquelle Y est le groupe chloro est obtenu sous forme d'une solution toluénique soumise à un séchage azéotropique.

**11.** Procédé selon l'une quelconque des revendications 7 à 10, dans lequel le composé de formule III est le N-méthoxy-N-méthylchloracétamide.

**12.** Procédé selon l'une quelconque des revendications 9 à 11, dans lequel le composé de formule III dans laquelle Y est le groupe chloro est en outre mis à réagir avec un réactif organométallique dans un procédé selon la revendication 1.

**13.** Procédé selon la revendication 1

- où A est le groupe 3,4-méthylènedioxyphényle ou phényléthynyle, et où M est Li ou MgBr quand A est le groupe 3,4-méthylènedioxyphényle et M est Li quand A est le groupe phényléthynyle ;
- le composé de formule III est le N-méthoxy-N-méthylchloracétamide, et le composé de formule III' est

$$HNCH_3(OCH_3)\bullet G.$$